# EUROPÄISCHE PATENTSCHRIFT

(11) **EP 2 170 273 B1**
(45) Veröffentlichungstag und Bekanntmachung des Hinweises auf die Patenterteilung: **26.11.2014**
(21) Anmeldenummer: 08760352.8
(22) Anmeldetag: 02.06.2008
(51) Int. Cl.: A61K 9/00, A61K 9/20, A61K 9/16

(54) **PHARMAZEUTISCHE FORMULIERUNG FÜR DIE HERSTELLUNG VON SCHNELL ZERFALLENDEN TABLETTEN**
PHARMACEUTICAL FORMULATION FOR THE PRODUCTION OF RAPIDLY DISINTEGRATING TABLETS
PRÉPARATION PHARMACEUTIQUE POUR PRODUIRE DES COMPRIMÉS À DÉLITEMENT RAPIDE

(30) Priorität: 06.06.2007 EP 07109725
(43) Veröffentlichungstag der Anmeldung: 07.04.2010
(73) Patentinhaber: BASF SE, 67056 Ludwigshafen (DE)
(72) Erfinder: KOLTER, Karl, 67117 Limburgerhof (DE); SCHÖNHERR, Michael, 67227 Frankenthal (DE); GEBERT, Silke, 67269 Grünstadt (DE); MEYER-BÖHM, Kathrin, 90537 Feucht (DE); MASCHKE, Angelika, 68159 Mannheim (DE)
(86) Internationale Anmeldenummer: PCT/EP2008/056765
(87) Internationale Veröffentlichungsnummer: WO 2008/148733

(56) Entgegenhaltungen:
- WO-A-98/22094
- WO-A-03/041683
- WO-A-2007/071581
- US-A1- 2002 071 864
- US-A1- 2005 244 343
- US-A1- 2005 244 492

## Beschreibung

Die vorliegende Erfindung betrifft pharmazeutische Formulierungen in Form von Agglomeraten für die Herstellung von schnell zerfallenden Tabletten, enthaltend Zucker oder Zuckeralkohole, Sprengmittel und wasserunlösliche Polymere.

Im Mund schnell zerfallende und/oder sich schnell auflösende Tabletten gewinnen für die orale Applikation von Arzneistoffen immer größere Bedeutung. Solche Tabletten müssen innerhalb kurzer Zeit, am besten innerhalb von 30 Sekunden in der Mundhöhle zerfallen, angenehm schmecken und dürfen kein sandiges Gefühl hinterlassen. Ferner sollen sie einfach herstellbar sein, wobei die Direkttablettierung erhebliche Vorteile gegenüber der Feuchtgranulation bietet, und eine hohe mechanische Festigkeit besitzen, damit sie Verpackungsprozeduren, Transporte und auch das Herausdrücken aus Verpackungen unbeschadet überstehen.

Die bisher beschriebenen Produkte und Verfahren erfüllen diese Anforderungen nicht oder nur sehr unzureichend.

Schnell zerfallende Tabletten bestehen häufig aus Zucker und Zuckeralkoholen, Brausesystemen, mikrokristalliner Cellulose und anderen nicht wasserlöslichen Füllstoffen Calciumhydrogenphosphat, Cellulose-Derivaten, Maisstärke, oder Polypeptiden. Weiterhin kommen wasserlösliche Polymere, übliche Sprengmittel (quervernetztes PVP, Na- und Ca-Salz der quervernetzten Carboxymethylcellulose, Na-Salz der Carboxymethylstärke, niedrigsubstituierte Hydroxypropylcellulose L-HPC) und im wesentlichen anorganische wasserunlösliche Bestandteile (Kieselsäuren, Silikate, anorganische Pigmente) zum Einsatz. Weiterhin können die Tabletten auch Tenside enthalten.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/0071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung JP 2004-265216 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

Die internationale Anmeldung WO03041683 offenbart Mundtabletten enthaltend a) 30 - 90% Mannit, b) 3 - 15% eines Sprengmittels wie z. B. Croscarmellose, c) vorzugsweise weniger als 10 % eines wasserunlöslichen Polymers wie z.B. Ethylcellulose als Überzugsmaterial.

Die US2005244343 und US2005244492 offenbaren Mundtabletten enthaltend a) 27 - 66,25 % eines Zuckeralkohols wie z.B. Mannit oder Erythriol, b) 5 -10 % eines Sprengmittels ausgewählt aus Stärkederivate, Croscarmellose. Statt eines wasserunlöslichen Polymers enthalten sie Silikate bzw. Titandioxide.

Die nachveröffentlichte internationale Anmeldung WO 2007/071581 offenbart Zubereitungen enthaltend 60-97% Zucker, 1-25% PVP und 1-15% wasserunlösliche filmbildende Polymere.

Aufgabe der vorliegenden Erfindung war es, im Mund schnell zerfallende Tabletten zu finden, die ein angenehmes Mundgefühl hinterlassen und mechanisch sehr stabil sind.

Demgemäß wurde eine pharmazeutische Zubereitung für die Herstellung von im Mund schnell zerfallenden Tabletten gefunden, welche aus Agglomeraten enthaltend
a) 60 - 97 Gew.-% mindestens eines Zuckers oder Zuckeralkohols oder Mischungen davon,
b) 1 - 25 Gew.-% eines Sprengmittels ausgewählt aus der Gruppe bestehend aus Croscarmellose, quervernetzter Natriumcarboxymethylstärke und L-Hydroxypropylcellulose,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,
besteht, wobei die Summe der Komponenten a) bis e) 100 Gew.-% beträgt.

Weiterhin wurde ein Verfahren zur Herstellung solcher Agglomerate gefunden.

Weiterhin wurden im Mund schnell zerfallende Tabletten, enthaltend solche Zubereitungen, gefunden. Die Tabletten zerfallen im Mund oder in wässrigem Milieu innerhalb von 40 Sekunden, bevorzugt innerhalb von 30 Sekunden, besonders bevorzugt innerhalb von 20 Sekunden. "Die Tabletten weisen bei 37 °C in Phospatpuffer, pH 7,2, eine Zerfallszeit von < 60 Sekunden auf.Die Zerfallszeit wird im Zerfallstester nach USP oder Pharm. Eur. bestimmt."

Die pharmazeutischen Zubereitungen enthalten als Komponente a) 60 bis 97 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 µm. Gewünschtenfalls können die Teilchengrößen durch Mahlen eingestellt werden. Bevorzugte Teilchengrößen liegen bei 30 bis 50 µm. Es kann sich aber auch empfehlen Teilchengrößen kleiner 30 µm zu verwenden. Ebenso kann es sich empfehlen, Zucker beziehungsweise Zuckeralkohole einzusetzen, die Mischungen von Fraktionen mit unterschiedlicher Partikelgröße enthalten, bespielsweise Mischungen aus 30 bis 70 gew.-% einer Korngrößenfraktion mit einer mittleren Teilchengröße von < 30 µm und 30 bis 70 Gew.-% einer Korngrößenfraktion mit einer mittleren Teilchengröße von 30 bis 50 µm. Bevorzugt werden Mannit, Erythrit oder Mischungen davon eingesetzt.

Als Komponente b) werden Sprengmittel in Mengen von 1 bis 25 Gew.-%, bevorzugt, 2 bis 15 Gew.-%, besonders bevorzugt 3 bis 10 Gew.-%, eingesetzt. Solche Sprengmittel sind wasserunlöslich, aber nicht filmbildend. Als Sprengmittel eignet sich Croscarmellose, eine quervernetzte Carboxymethyllcellulose, wobei als Croscarmellose erfindungsgemäß auch deren Natrium- und Calciumsalze gemeint sind. Weiterhin eignet sich Natriumcarboxymethylstärke. Ebenso eignet sich L-Hydroxypropylcellulose, bevorzugt mit 5 bis 16% Hydroxypropoxy-gruppen.

Als Komponente c) werden wasserunlösliche Polymere eingesetzt in Mengen von 1 bis 15 Gew.-%, bevorzugt 1 bis 10 Gew.-%, eingesetzt. Dabei handelt es sich um Polymere. Bevorzugt sind Polymere, die im pH-Bereich von 1 bis 14 unlöslich sind, also eine bei jedem pH-Wert pH-unabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrytat-Methylmethacrylat-Dimethylaminoethylmethacrylat-Terpolymere

Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E.

Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton.

Weiterhin können die Formulierungen als Komponenten d) wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere, Hydroxypropylmethylcellulose, Hydroxypropylcellulose, Hydroxyethylcellulose, Carragenane, Pektine, Xanthane, Alginate. Gewünschtenfalls können durch Zusatz von pharmazeutisch üblichen Hilfsstoffen (Komponenten e)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden. Folgende Stoffe sind hierbei besonders geeignet: Citronensäure, Weinsäure, Ascorbinsäure, Natriumdihydrogenphosphat, Cyclamat, Saccharin-Na, Aspartam, Menthol, Pfefferminzaroma, Fruchtaromen, Vanillearoma, Glutamat, Riboflavin, Betacarotin, wasserlösliche Farbstoffe, feinteilige Farblacke.

Durch Zusatz von Verdickungsmitteln wie hochmolekularen Polysacchariden kann das Mundgefühl durch Erhöhung der Weichheit und des Volumengefühls zusätzlich verbessert werden.

Weiterhin können als Komponenten e) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine.

Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Die Herstellung der erfindungsgemäßen Formulierungen kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschliessend getrocknet. Gemäß der vorliegenden Erfindung wird als Granulierflüssigkeit eine wässrige Dispersion der Komponente c), des wasserunlöslichen Polymers, eingesetzt.

Bei der Agglomeration in der Wirbelschicht wird eine wässrige Dispersion des wasserunlöslichen Polymers auf eine wirbelnde Mischung aus Zucker oder Zuckeralkohol und quervernetztem PVP gesprüht, wodurch die feinen Teilchen agglomerieren. Die Zulufttemperaturen betragen 30 bis 100°C, die Ablufttemperaturen 20 bis 70°C.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von quervernetztem PVP und die Eindüsung einer wässrigen Dispersion des wasserunlöslichen Polymeren, wodurch die Teilchen agglomerieren. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Für den Agglomerationsprozess kann es sich als günstig erweisen, einen mehrstufigen Sprühprozess zu fahren. Zu Anfang wird die Sprührate niedrig gehalten, um ein Überfeuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder -temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanischen Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin kann die Agglomeration auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das quervenetzte PVP in der wässrigen Dispersion des wasserunlöslichen Polymers suspendiert.

Die so erhaltenen Agglomerate weisen eine mittlere Teilchengröße von 100 - 600 µm, bevorzugt 120 - 500 µm und besonders bevorzugt von 140 - 400 µm auf.

Das wasserunlösliche, filmbildende Polymer dient dabei als Agglomerationsmittel, um die feinen Zucker oder Zuckeralkoholkristalle und die Teilchen von Sprengmittel zu agglomerieren.

Die erfindungsgemäßen Formulierungen können vorteilhaft auch für die Herstellung von Tabletten verwendet werden, die vor der Anwendung in einem Glas Wasser zerfallen gelassen werden. Auch die Herstellung von Tabletten, die intakt geschluckt werden, ist natürlich möglich.

Für die Herstellung von Tabletten können die üblichen Verfahren verwendet werden, wobei die Direkttablettierung und die Walzenkompaktierung besondere Vorteile bieten. Aufgrund der besonderen Eigenschaften der erfindungsgemäßen Formulierungen werden in der Regel nur Wirkstoff, erfindungsgemäße Formulierung und ein Schmiermittel benötigt. Die Tablettenrezeptur ist somit sehr einfach, sehr reproduzierbar und das Verfahren leicht zu validieren.

Die erfindungsgemäßen Granulate eignen sich grundsätzlich zur Verarbeitung mit allen Wirkstoffen. Insbesondere eignen sich die Granulate für die Herstellung von Arzneiformen mit der im Folgenden genannten Dosierung des jeweiligen Wirkstoffs, wobei der Wirkstoff auch geschmacksmaskiert eingesetzt werden kann:
Zolmitriptan 2,5 mg, Rizatriptan 5 mg, Diphenhydramin-HCl (geschmacksmaskiert) 20 mg, Brompheniramin 5 mg, Chlorpheniramin 5 mg, Pseudoephedrin (geschmacksmaskiert) 30mg,
Paracetamol (geschmacksmaskiert) 250 mg, Ibuprofen (geschmacksmaskiert) 200 mg, Acetylsalicylsäure 250 mg (geschmacksmaskiert), Hyoscyaminsulfat 0,125 mg, Mirtazapin 15 mg,
Selegelin-HCl 1,25 mg, Ondansetron 4 mg, Olanzapin 5 mg, Clonazepam 1 mg, Cetirizindihydrochlorid 10 mg, Desloratadin 5 mg, Enalaprilmaleat 5 mg, Domperidonmaleat 10 mg, Scopolamin 0,25 mg,
Oxazepam 15 mg, Lorazepam 2,5 mg, Clozapin 25 mg, Dihydroergotaminmesylat 5 mg,
Nicergolin 5 mg, Phloroglucinol 80 mg, Metopimazin 7,5 mg, Triazolam 0,5 mg, Protizolam 0,5 mg,
Tramadol 50 mg, Zolpidemtartrat 5 mg, Cisapride 5mg, Risperidon 2 mg, Azithromycin 100 mg (geschmacksmaskiert), Roxithromycin 50 mg (geschmacksmaskiert), Clarithromycin 125 mg (geschmacksmaskiert), Erythromycinestolat 250 mg (geschmacksmaskiert),
Apomorphin 20 mg, Fentanyl 0,6 mg,

Überraschenderweise wurde gefunden, dass ein wasserunlösliches filmbildendes Polymer den Zerfall von Tabletten erheblich beschleunigt. Dies ist umso überraschender, da solche Polymere in der Regel für die Herstellung von retardierten Arzneiformen eingesetzt werden, die innerhalb mehrerer Stunden nicht zerfallen. Die Zerfallszeiten unter Verwendung von Polyvinylacetat als wasserunlöslichem Polymer sind erheblich kürzer als bei wasserlöslichen Polymeren.

Ferner besitzen die erfindungsgemäßen Zubereitungen außergewöhnlich gute Fließfähigkeiten und Verpressbarkeiten, die zu mechanisch sehr stabilen Tabletten führen.

Die Bruchfestigkeit der mit Hilfe der erfindungsgemäßen pharmazeutischen Formulierungen erzeugten Tabletten beträgt > 50 N. Häufig liegen die Bruchfestigkeiten oberhalb von 80 N, selbst unter Verwendung schwer pressbarer Wirkstoffe. Die Friabilitäten betragen < 0,2 %. Beschädigungen beim üblichen Tablettenhandling treten somit nicht auf.

### Beispiele

Die Agglomerate wurden in der Wirbelschicht (GPCG 3.1, Glatt) mittels Top- SprayVerfahren hergestellt: Zuckeralkohol und Sprengmittel (Croscarmellose, quervernetzte Natriumcarboxymethylstärke, L-Hydroxypropylcellulose) wurden vorgelegt und mit wässriger Bindemitteldispersion agglomeriert. Als wässrige Bindemitteldispersion wurde eine kommerziell erhältliche Polyvinylacetatdispersion (Kollicoat® SR30 D, Fa. BASF AG) verwendet. Als L-Hydroxypropylcellulose wurde eine Type mit einem Hydroxypropoxy-Gehalt von 11 % verwendet.

**Tabelle 1: Rezepturzusammensetzung in Gew.%**

| | A | B | C | D | E |
|---|---|---|---|---|---|
| Mannitol (d_{0,5}:36µm] | 90 | 90 | 90 | 44 | 76 |
| Erythritol (d_{0,5}:44µm] | | | | 44 | |
| Sorbitol (d_{0,5}:47µm] | | | | | 11 |
| Croscarmellose-Na | 5 | | | | 4 |
| Croscarmellose-Ca | | | | 8 | |
| quervernetzte Natriumcarboxymethylstärke | | 5 | | | 3 |
| L-Hydroxypropylcellulose | | | 5 | | |
| Kollicoat SR30D (Feststoff) | 5 | 5 | 5 | 4 | 6 |

In einem zweistufigen Agglomerationsprozess, bei dem zunächst eine geringere Sprührate gewählt wurde und dann die Sprührate erhöht wurde, wurden folgende Herstellungsparameter angewendet:

| | |
|---|---|
| Ansatzgröße: | 0,6 kg |
| Konzentration der Bindemitteldispersion: | 10 Gew.% Feststoff |

**Tabelle 2: Herstellungsbedingungen der Formulierungen**

| | A | B | C | D | E |
|---|---|---|---|---|---|
| Zulufttemperatur [°C] | 49 | 52 | 52 | 51 | 53 |
| Ablufttemperatur [°C] | 25 | 28 | 31 | 27 | 31 |
| Sprührate | 15g/min (10min)/ Rest 20g/min | 15g/min (10min)/ Rest 20g/min | 15g/min (10min)/ Rest 20g/min | 15g/min (10min)/ Rest 20g/min | 15g/min (10min)/ Rest 20g/min |

Die so hergestellten Agglomerate wurden mit 2,0 Gew.% Schmiermittel (Mg-Stearat) bezogen auf die zu verpressende Mischung im Turbulamischer gemischt und auf einer vollinstrumentierten Excenterpresse (Korsch XP1) bei 30Hüben/min zu Tabletten verarbeitet.

Zur Tablettierung wurde die Excenterpresse mit einem 10mm- Stempel (biplan, facettiert) ausgestattet und die Presskraft so eingestellt, dass Tabletten mit einer Bruchfestigkeit von 40-60N resultierten.

Die Tabletten wurden hinsichtlich Bruchfestigkeit (Tablettentester HT-TMB-CI-12 F, Fa. Kraemer), Zerfallszeit in Phosphatpuffer pH 7,2 (Zerfallstester ZT 74, Erweka) und Freisetzungsgeschwindigkeit in Magensaft (Freisetzungsgerät, Erweka) untersucht.

**Tabelle 3: Tabletteneigenschaften**

| | Tablettierdaten | Tablettenparameter | |
|---|---|---|---|
| | Presskraft [kN] | Bruchfestigkeit [N] | Zerfallszeit [s] |
| A | 4,0 | 49 | 15 |
| B | 3,7 | 51 | 21 |
| C | 3,8 | 40 | 53 |
| D | 5,3 | 52 | 38 |
| E | 4,6 | 51 | 32 |

## Patentansprüche

1. Pharmazeutische Formulierung in Form von Agglomeraten enthaltend
a) 60 - 97 Gew.-% Zucker oder Zuckeralkohole,
b) 1 - 25 Gew.-% eines Sprengmittels ausgewählt aus der Gruppe bestehend aus Croscarmellose, Natriumcarboxymethylstärke und L-Hydroxypropylcellulose,
c) 1 - 15 Gew.-% wasserunlösliche, filmbildende Polymere
d) 0 -15 Gew.-% wasserlösliche Polymere, und
e) 0 -15 Gew.-% weitere pharmazeutisch übliche Hilfsstoffe
f) wobei die Summe der Komponenten a) bis e) 100 Gew.-% beträgt.

2. Formulierung nach Anspruch 1 **dadurch gekennzeichnet, dass** die mittlere Teilchengröße der Agglomerate 100 µm bis 600 µm beträgt.

3. Formulierung nach Anspruch 1 oder 2, enthaltend als Zuckeralkohol Mannit oder, Erythrit oder Mischungen davon.

4. Formulierung nach einem der Ansprüche 1 bis 3, enthaltend ein Croscarmellose als Natrium- oder Calcium-Salz.

5. Formulierung nach einem der Ansprüche 1 bis 4, enthaltend eine L-Hydroxypropylcellulose mit 5 bis 16% Hydroxypropoxy-Gruppen.

6. Formulierung nach einem der Ansprüche 1 bis 5, wobei als wasserunlösliches filmbildendes Polymer Polyvinylacetat verwendet wird.

7. Formulierung nach einem der Ansprüche 1 bis 6, wobei das wasserunlösliche filmbildende Polymer Polyvinylacetat in Form einer wässrigen Dispersion eingesetzt wird.

8. Formulierung nach einem der Ansprüche 1 bis 7 , wobei als wasserlösliche Polymer Polyvinylpyrrolidon verwendet wird.

9. Formulierung nach einem der Ansprüche 1 bis 8, wobei als weitere pharmazeutisch übliche Stoffe Säuerungsmittel, Süßstoff, Aromen, Geschmacksverstärker, Farbstoffe, Verdickungsmittel, Tenside und feinteilige Pigmente verwendet werden.

10. Formulierung nach einem der Ansprüche 1 bis 9, enthaltend Agglomerate aus
a) 70 - 95 Gew.-% Zucker oder Zuckeralkoholen
b) 2 - 15 Gew.-% eines Sprengmittels ausgewählt aus der Gruppe bestehend aus Croscarmellose, Natriumcarboxymethylstärke und L-Hydroxypropylcellulose,
c) 1 - 10 Gew.% wasserunlösliche, filmbildende Polymere
d) 0 - 2 Gew.-% wasserlösliches Polyvinylpyrrolidon, und
e) 0 - 15 Gew.-% weitere pharmazeutisch übliche Hilfsstoffe.

11. Formulierung nach einem der Ansprüche 1 bis 10, enthaltend Agglomerate aus
a) 75 - 95 Gew.-% Mannit oder Erythrit oder einer Mischung davon,
b) 3 - 10 Gew.-% eines Sprengmittels ausgewählt aus der Gruppe bestehend aus Croscarmellose, Natriumcarboxymethylstärke und L-Hydroxypropylcellulose,
c) 1 - 10 Gew.-% Polyvinylacetat,
d) 0 - 2 Gew.-% wasserlösliches Polyvinylpyrrolidon, und
e) 0 -15 Gew.-% weitere pharmazeutisch übliche Hilfsstoffe

12. Verwendung der pharmazeutischen Formulierung nach einem der Ansprüche 1 bis 11 zur Herstellung von Tabletten,.

13. Verwendung der pharmazeutischen Formulierung nach Anspruch 12 zur Herstellung von im Mund schnell zerfallenden Tabletten,.

14. Verwendung nach Anspruch 12 oder 13, wobei die Tabletten eine Bruchfestigkeit größer 50 N aufweisen.

15. Verwendung nach einem der Ansprüche 12 bis 14, wobei die Tabletten 20 bis 99 Gew.-%, bezogen auf das gesamte Tablettengewicht, der pharmazeutischen Formulierung enthalten.

16. Verwendung nach einem der Ansprüche 12 bis 15, wobei die Tabletten Schmiermittel als weitere Hilfsstoffe enthalten.

17. Verfahren zur Herstellung einer pharmazeutischen Formulierung gemäß einem der Ansprüche 1 bis 9, **dadurch gekennzeichnet, dass** feinteilige Zucker oder Zuckeralkoholteilchen und Sprengmittel mit einer wässrigen Dispersion des wasserunlöslichen Polymers agglomeriert werden.

18. Verfahren nach Anspruch 17, **dadurch gekennzeichnet, dass** feinteilige Zucker-oder Zuckeralkoholteilchen mit einer wässrigen Dispersion des wasserunlöslichen Polymers, die zusätzlich Sprengmittel suspendiert enthält, agglomeriert werden.

19. Verfahren nach Anspruch 17 oder 18, **dadurch gekennzeichnet, dass** die Agglomeration in einem Wirbelschichtgranulator, einem Mischer oder einem Sprühturm erfolgt.

## Claims

1. A pharmaceutical formulation in the form of agglomerates comprising
a) 60-97% by weight of sugar or sugar alcohols,
b) 1-25% by weight of a disintegrant selected from the group consisting of croscarmellose, sodium carboxymethylstarch and L-hydroxypropylcellulose,
c) 1-15% by weight of water-insoluble, film-forming polymers
d) 0-15% by weight of water-soluble polymers and
e) 0-15% by weight of further pharmaceutically customary excipients
f) the total of the components a) to e) being 100% by weight.

2. The formulation according to claim 1, wherein the average particle size of the agglomerates is from 100 µm to 600 µm.

3. The formulation according to claim 1 or 2, comprising mannitol or erythritol or mixtures thereof as sugar alcohol.

4. The formulation according to any of claims 1 to 3, comprising a croscarmellose as sodium or calcium salt.

5. The formulation according to any of claims 1 to 4, comprising an L-hydroxypropylcellulose having 5 to 16% hydroxypropoxy groups.

6. The formulation according to any of claims 1 to 5, polyvinyl acetate being used as water-insoluble film-forming polymer.

7. The formulation according to any of claims 1 to 6, where the water-insoluble film-forming polymer polyvinyl acetate is employed in the form of an aqueous dispersion.

8. The formulation according to any of claims 1 to 7, where polyvinylpyrrolidone is used as water-soluble polymer.

9. The formulation according to any of claims 1 to 8, where acidifiers, sweeteners, flavors, flavor enhancers, colorants, thickeners, surfactants and finely divided pigments are used as further pharmaceutically customary substances.

10. The formulation according to any of claims 1 to 9, comprising agglomerates composed of
a) 70-95% by weight of sugar or sugar alcohols
b) 2-15% by weight of a disintegrant, selected from the group consisting of croscarmellose, sodium carboxymethylstarch and L-hydroxypropylcellulose,
c) 1-10% by weight of water-insoluble, film-forming polymers
d) 0-2% by weight of water-soluble polyvinylpyrrolidone, and
e) 0-15% by weight of further pharmaceutically customary excipients.

11. The formulation according to any of claims 1 to 10, comprising agglomerates composed of
a) 75-95% by weight of mannitol or erythritol or a mixture thereof,
b) 3-10% by weight of a disintegrant selected from the group consisting of croscarmellose, sodium carboxymethylstarch and L-hydroxypropylcellulose,
c) 1-10% by weight of polyvinyl acetate,
d) 0-2% by weight of water-soluble polyvinylpyrrolidone, and
e) 0-15% by weight of further pharmaceutically customary excipients.

12. The use of the pharmaceutical formulation according to any of claims 1 to 11 for producing tablets.

13. The use of the pharmaceutical formulation according to claim 12 for producing tablets which disintegrate rapidly in the mouth.

14. The use according to claim 12 or 13, where the tablets have a hardness greater than 50 N.

15. The use according to any of claims 12 to 14, where the tablets comprise from 20 to 99% by weight, based on the total tablet weight, of the pharmaceutical formulation.

16. The use according to any of claims 12 to 15, where the tablets comprise lubricants as further excipients.

17. A process for producing a pharmaceutical formulation according to any of claims 1 to 9, wherein finely divided sugar or sugar alcohol particles and disintegrant are agglomerated with an aqueous dispersion of the water-insoluble polymer.

18. The process according to claim 17, wherein finely divided sugar or sugar alcohol particles are agglomerated with an aqueous dispersion of the water-insoluble polymer, which additionally comprises suspended disintegrant.

19. The process according to claim 17 or 18, wherein the agglomeration takes place in a fluidized bed granulator, a mixer or a spray tower.

## Revendications

1. Formulation pharmaceutique sous forme d'agglomérats contenant
a) 60-97% en poids de sucre ou d'alcools de sucre,
b) 1-25% en poids d'un agent de désintégration choisi dans le groupe constitué par la croscarmellose, le carboxyméthylamidon de sodium et la L-hydroxypropylcellulose,
c) 1-15% en poids de polymères filmogènes insolubles dans l'eau
d) 0-15% en poids de polymères solubles dans l'eau, et
e) 0-15% en poids d'autres adjuvants pharmaceutiquement usuels,
f) la somme des composants a) à e) valant 100% en poids.

2. Formulation selon la revendication 1, **caractérisée en ce que** la grosseur moyenne des agglomérats est de 100 µm à 600 µm.

3. Formulation selon la revendication 1 ou 2, contenant, comme alcool de sucre, du mannitol, de l'érythritol ou des mélanges de ceux-ci.

4. Formulation selon l'une quelconque des revendications 1 à 3, contenant une croscarmellose sous forme de sel de sodium ou de calcium.

5. Formulation selon l'une quelconque des revendications 1 à 4, contenant une L-hydroxypropylcellulose présentant 5 à 16% de groupes hydroxypropoxy.

6. Formulation selon l'une quelconque des revendications 1 à 5, du poly(acétate de vinyle) étant utilisé comme polymère filmogène insoluble dans l'eau.

7. Formulation selon l'une quelconque des revendications 1 à 6, le polymère filmogène insoluble dans l'eau, le poly(acétate de vinyle), étant utilisé sous forme d'une dispersion aqueuse.

8. Formulation selon l'une quelconque des revendications 1 à 7, de la polyvinylpyrrolidone étant utilisée comme polymère soluble dans l'eau.

9. Formulation selon l'une quelconque des revendications 1 à 8, des agents acidifiants, des édulcorants, des arômes, des exhausteurs de goût, des colorants, des épaississants, des agents tensioactifs et des pigments finement divisés étant utilisés comme autres substances pharmaceutiquement usuelles.

10. Formulation selon l'une quelconque des revendications 1 à 9, contenant des agglomérats constitués de
a) 70-95% en poids de sucre ou d'alcools de sucre
b) 2-15% en poids d'un agent de désintégration choisi dans le groupe constitué par la croscarmellose, le carboxyméthylamidon de sodium et la L-hydroxypropylcellulose,
c) 1-10% en poids de polymères filmogènes insolubles dans l'eau
d) 0-2% en poids de polyvinylpyrrolidone soluble dans l'eau, et
e) 0-15% en poids d'autres adjuvants pharmaceutiquement usuels.

11. Formulation selon l'une quelconque des revendications 1 à 10, contenant des agglomérats constitués de
a) 75-95% en poids de mannitol ou d'érythritol ou d'un mélange de ceux-ci,
b) 3-10% en poids d'un agent de désintégration choisi dans le groupe constitué par la croscarmellose, le carboxyméthylamidon de sodium et la L-hydroxypropylcellulose,
c) 1-10% en poids de poly(acétate de vinyle),
d) 0-2% en poids de polyvinylpyrrolidone soluble dans l'eau, et
e) 0-15% en poids d'autres adjuvants pharmaceutiquement usuels,

12. Utilisation de la formulation pharmaceutique selon l'une quelconque des revendications 1 à 11 pour la préparation de comprimés.

13. Utilisation de la formulation pharmaceutique selon la revendication 12 pour la préparation de comprimés à désintégration rapide en bouche.

14. Utilisation selon la revendication 12 ou 13, les comprimés présentant une résistance à la rupture supérieure à 50 N.

15. Utilisation selon l'une quelconque des revendications 12 à 14, les comprimés contenant 20 à 99% en poids, par rapport au poids total des comprimés, de la formulation pharmaceutique.

16. Utilisation selon l'une quelconque des revendications 12 à 15, les comprimés contenant des lubrifiants comme autres adjuvants

17. Procédé pour la préparation d'une formulation pharmaceutique selon l'une quelconque des revendications 1 à 9, **caractérisé en ce qu'**on agglomère du sucre finement divisé ou des particules d'alcool de sucre et l'agent de désintégration avec une dispersion aqueuse du polymère insoluble dans l'eau.

18. Procédé selon la revendication 17, **caractérisé en ce qu'**on agglomère des particules de sucre ou des particules d'alcool de sucre finement divisées avec une dispersion aqueuse du polymère insoluble dans l'eau qui contient en outre l'agent de désintégration en suspension.

19. Procédé selon la revendication 17 ou 18, **caractérisé en ce que** l'agglomération est réalisée dans un granulateur à lit fluidisé, un mélangeur ou une tour de pulvérisation.
